# EUROPÄISCHE PATENTSCHRIFT

(11) **EP 0 871 404 B1**
(45) Veröffentlichungstag und Bekanntmachung des Hinweises auf die Patenterteilung: **05.07.2000**
(21) Anmeldenummer: 96919526.2
(22) Anmeldetag: 11.04.1996
(51) Int. Cl.: A61B 18/12

(54) **VORRICHTUNG ZUR LINIENFÖRMIGEN HOCHFREQUENZ-KATHETERABLATION ENDOMYOKARDIALEN GEWEBES**
DEVICE FOR THE LINEAR HIGH-FREQUENCY CATHETER ABLATION OF ENDOMYOCARDIAL TISSUE
PROCEDE D'ABLATION LINEAIRE PAR CATHETER HAUTE FREQUENCE D'UN TISSU ENDOMYOCARDIQUE

(30) Priorität: 14.12.1995 DE 29519651 U
(43) Veröffentlichungstag der Anmeldung: 21.10.1998
(73) Patentinhaber: Bip Acquisition Company, Inc., Wilmington, De. (US)
(72) Erfinder: Muntermann,Axel, 355583 Wetzlar (DE)
(74) Vertreter: Marsh, Roy David
(86) Internationale Anmeldenummer: DE9600638
(87) Internationale Veröffentlichungsnummer: WO9721387

(56) Entgegenhaltungen:
- EP-A- 0 499 491
- WO-A-93/08755

## Beschreibung

Die Erfindung betrifft eine Vorrichtung zur Hochfrequenz- und insbesondere zur Radiofrequenz- und/oder Mikrowellen-Katheterablation endomyokardialen Gewebes sowie einen entsprechenden Ablationskatheter.

Zur Behandlung von Herzrhythmusstörungen, insbesondere von durch endomyokardialem Gewebe hervorgerufenen Störungen erwies sich das thermische Veröden von Rhythmusstörstellen in der Oberfläche des Herzmuskels als erfolgreich. Hierbei wurde ein Ablationskatheter kontrolliert in das Herz des Patienten eingeführt und mit einer im wesentlichen punktförmigen Elektrode in der Oberfläche des Muskels, somit im Bereich des für die Reizleitung verantwortlichen Gewebes eine lokale Koagulation durchgeführt. Üblicherweise wurde hierbei durch einen Hochfrequenzgenerator mit Frequenzen von 300 kHz bis 700 kHz eine Koagulationsnarbe bis zu einer Tiefe von 3 bis 5 mm erzeugt. Nachteilig ist diesem Verfahren jedoch, daß zur Durchführung einer Behandlung der Ablationskatheter mehrere Male im Bereich der Behandlungsstelle neu zu plazieren ist und sich somit der Behandlungsvorgang zeitaufwendig und arbeitsintensiv gestaltet.

Die WO 93/08755 beschreibt einen derartigen Katheter zur punktförmigen Katheterablation.

Aus der EP-A-0 499 491 A2 ist ein Katheter mit einer Vielzahl von expandierbaren Elektroden bekannt, jedoch wird die Verwendung von thermischen Sensoren dort nicht beschrieben.

Der Erfindung liegt die Aufgabe zugrunde, die Katheterablation endomyokardialen Gewebes zu vereinfachen und den hierzu notwendigen Zeit- und Behandlungsbedarf zu vermindern.

In überraschend einfacher Weise wird diese Aufgabe bereits durch eine Hochfrequenz-Katheterablationsvorrichtung gemäß Anspruch 4 sowie einen Hochfrequenz-Ablationskatheter gemäß Anspruch 1 gelöst.

Durch den Einsatz von mehrere Elektroden umfassenden Kathetern mit selektiv ansteuerbaren Elektroden kann erstmalig eine linienförmige Ablation in der Oberfläche des Herzmuskels bei einer einmaligen Anwendung durchgeführt werden.

Durch die linienförmige Anordnung der Elektroden kann darüber hinaus sichergestellt werden, daß eine bereichsweise und nicht nur punktförmige Unterbrechung der Reizleitung erhalten wird und somit die Wirkung von Störstellen sicherer als bei den bisherigen Behandlungsverfahren ausgeschlossen werden kann. Durch die kürzere Behandlungszeit vermindert sich die Belastung des Patienten wesentlich, welchem insbesondere bei Risikopatienten starke Bedeutung zukommt und die erfindungsgemäße Vorrichtung zur intensivmedizinischen und stationären Notfallbehandlung geeignet macht.

Als vorteilhaft hat es sich erwiesen, Hochfrequenzgeneratoren mit Leistungen von bis zu 200 Watt und mehr oder handelsübliche Hochfrequenz-Ablationsgeräte zusammen mit den erfindungsgemäßen Vorrichtungen zu verwenden. Die Erfindung ist jedoch nicht auf festgelegte Leistungen des verwendeten Hochfrequenz-Ablationsgerät beschränkt und kann im wesentlichen mit jedem Hochfrequenz-Ablationsgerät verwendet werden.

Werden den Elektroden jeweils einige thermische Sensoren zugeordnet, mit welchen die Betriebstemperaturen der Elektroden erfaßbar sind, kann im Zusammenwirken mit einer Steuereinheit eine gezielte und dosierte linienförmige Koagulation erfolgen. Hierzu wird die Betriebstemperatur der jeweiligen Elektrode erfaßt und durch die Steuereinheit in deren zeitlichem Verlauf und absoluten Höhe eingestellt bzw. geregelt. Die erfindungsgemäße Vorrichtung kann im wesentlichen in drei verschiedenen Betriebszuständen vorteilhaft betrieben werden.

Dies sind der ungeregelte, der teilgeregelte und der vollständig geregelte Betriebszustand. Im ungeregelten Betriebszustand wird die Radiofrequenzenergie während fest vorgebbaren Zeitintervallen, vorzugsweise jeweils 10 mS an die jeweilige Elektrode des aktiven Katheterabschittes angelegt.

Im teilgeregelten Betriebszustand wird zwar die Temperatur der Elektroden erfasst, jedoch wird ein Temperatursensor jeweils für mehr als eine Elektrode verwendet. Bei bevorzugten Ausführungsformen der erfindungsgemäßen Vorrichtung wird jeweils ein Temperatursensor für entweder alle oder für jeweils zwei oder drei Ablationselektroden verwendet. Hierdurch läßt sich ein noch sehr elastischer Katheter mit gutem Regelverhalten verwirklichen.

Im vollständig geregelten Betriebszustand kann durch Verwendung jeweils eines Temperatursensors für jede Ablationselektrode auch unter kritischsten Gegebenheiten, etwa bei schwieriger Ankopplung an das zu behandelnde Gewebe stets die optimale Behandlungstemperatur bzw. die optimale Energieabgabe entlang des gesamten aktiven Katheterabschnitts erzielt werden.

Darüberhinaus kann die Messung der Impedanz der Katheterelektroden gegenüber einer am Patienten angeordneten indiferenten Elektrode Aufschluß über die richtige Lage des aktiven Katheterabschnitts relativ zu dem zu behandelnden Gewebe geben.

Es ist auch möglich, anstelle eines im Radiofrequenzbereich emittierenden Hochfrequenzgengerators ein im Mikrowellenbereich arbeitendes Gerät zu verwenden.

Zum Durchführen eines lokalen Koagulationsvorgangs haben sich Temperaturen von zwischen etwa 40 bis etwa 80°C, vorzugsweise 45, 50, 55 und 60 °C, als wirksam erwiesen. Nach Erreichen dieser Temperatur kann die vom Hochfrequenzgenerator oder Hochfrequenz-Ablationsgerät zugeführte Energie vermindert oder vorzugsweise der nächsten Elektrode oder einer Last, wie beispielsweise einem gekühlten Lastwiderstand, zugeführt werden.

Desweiteren kann in ebenfalls vorteilhafter Weise die Hochfrequenzenergie den Elektroden moduliert in Form fester Impulse mit sich ändernder Häufigkeit zugeführt werden. Hierbei hat es sich als vorteilhaft erwiesen, die Temperatur anfänglich entlang einer vorgebbaren zeitlich ansteigenden Bezugskurve zu erhöhen, wobei durch einen Ist/Sollwertvergleich der Bezugskurve mit dem momentanten Temperaturwert der Elektrode festgestellt wird, ob der Elektrode Energie in diesem oder in einem späteren Zyklus zugeführt werden soll. Hierdurch wird ein thermisches Überschießen über den Sollwert und damit einhergehende Patientenbeeinträchtigungen mit hoher Sicherheit ausgeschlossen.

Durch die Abführung der vom Generator gelieferten, jedoch momentan nicht benötigten Leistung an eine Last wird der Generator vor hohen Lastschwankungen bewahrt und kann seine Leistung unabhängig von äußeren Störungen gleichmäßiger zur Verfügung stellen.

Als besonders zweckmäßig erwiesen sich Elektroden, die entlang einer ununterbrochenen Linie mit einer Länge von bis zu etwa 7 cm jeweils durch Isolationsbereiche voneinander getrennt, vorzugsweise am Ende des Ablationskatheters angeordnet waren.

Die Behandlung wird weiterhin unterstützt und deren Sicherheit gefördert, wenn im teilgeregelten oder vollständig geregelten Betriebszustand die Temperatur einer jeweiligen Elektrode und die Zeitdauer der Energieabgabe an diese Elektrode an einer Anzeigeeinrichtung dargestellt wird.

Die Verbindung einer oder mehrerer Elektroden mit einem EKG-Monitor macht die lokale Herztätigkeit vor, während und nach der Behandlung erfaßbar und anzeigbar und liefert dem behandelnden Arzt somit sofortige Aussagen über deren Erfolg.

In zweckmäßiger Weise ist an einem Bedienfeld die Anzahl der zur Ablation benötigten Elektroden, deren Soll-Temperatur, zeitliche Energiezufuhr und/oder deren jeweiliger Betriebszustand einstellbar.

Die Erfindung wird nachfolgend anhand bevorzugter Ausführungsformen und unter Bezugnahme auf die beigefügten Zeichnungen im einzelnen beschrieben. Es zeigen:
- Fig. 1: eine schematische Darstellung der Vorrichtung zur linienförmigen Hochfrequenz-, insbesondere Radiofrequenz-Katheterablation,
- Fig. 2: eine vergrößerte Darstellung des Ablationskatheters mit jeweiligen, durch Isolationsbereiche voneinander getrennten Elektroden und
- Fig. 3: eine vergrößerte Darstellung einer weiteren Ausführungsform des Ablationskatheters mit jeweiligen, durch Isolationsbereiche voneinander getrennten Elektroden und mit Lichtleitern.

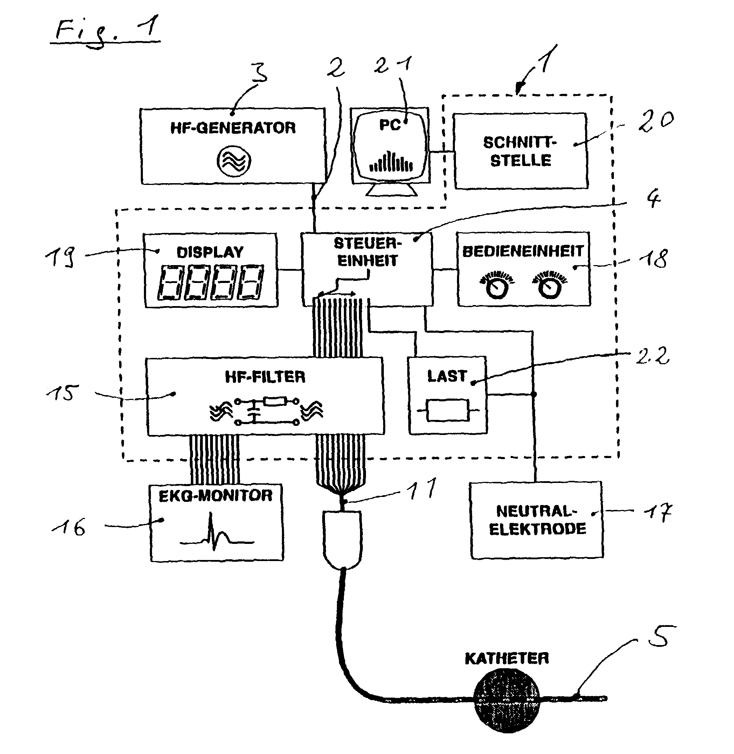

Nachfolgend wird auf Fig. 1 Bezug genommen, in welcher die im ganzen mit 1 bezeichnete Vorrichtung 2 in einer schematischen Blockdarstellung gezeigt ist.

Die erfindungsgemäße Vorrichtung 1 umfaßt einen Anschluß 2 an einem Hochfrequenzgenerator 3. Anstelle des Hochfrequenzgenerators 3 ist jedoch alternativ ebenfalls ein handelsübliches Hochfrequenz-Ablationsgerät 3 oder ein Mikrowellengenerator 3 verwendbar.

Der Frequenzbereich der Geräte 3 sollte im Falle der Radiofrequenz-Katheterablation von etwa 300 bis etwa 750 kHz oder höher reichen, und es sind Leistungen von weniger als 50 W, 120 W, 150 W, 200 W oder mehr verwendbar. Die Steuereinheit 4 verbindet den Katheter 5 auf die nachfolgend detaillierter beschriebene Weise mit dem HF-Generator 3 oder dem Hochfrequenz-Ablationsgerät 3.

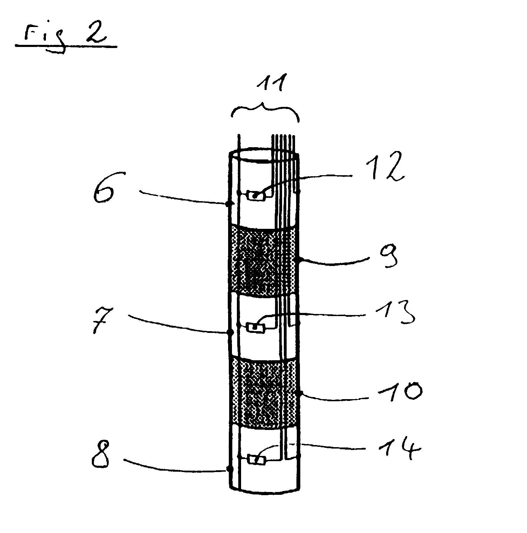

Der Katheter 5 umfaßt, wie in Fig. 2 detaillierter dargestellt ist, mehrere selektiv ansteuerbare Elektroden 6, 7 und 8, die durch Isolationsbereiche 9 und 10 voneinander elektrisch isoliert, jedoch mechanisch flexibel gehalten sind. Über Leitungen 11 sind bei Kathetern 1 für den vollständig geregelten Betriebszustand jeweils den Elektroden 6, 7 und 8 zugeordnete thermische Sensoren 12, 13 und 14 sowie die Elektroden 6, 7 und 8 mit der Steuereinheit 4 elektrisch verbunden.

Bei den in den Figuren nicht dargestellten Kathetern für den teilgeregelten Betriebszustand sind jeweils weniger thermische Sensoren 12, 13, und 14 als Elektroden 6, 7 und 8 vorhanden. Besonders bevorzugte Ausführungsformen dieses Kathetertyps weisen nur einen einzigen oder jeweils einen thermischen Sensor 12, 13, 14 für jeweils zwei oder drei Elektroden auf.

Die Katheter für den teilgeregelten Betrieb und den vollständig geregelten Betrieb sind ungeregelt betreibbar.

Ein HF-Filter 15 verbindet einen vorzugsweise extern angeordneten unipolaren oder bipolaren EKG-Monitor 16 je nach Ausführung der erfindungsgemäßen Vorrichtung entweder mit einer der Elektroden 6, 7 oder 8, verbindet die erfindungsgemäße Vorrichtung mit ausgewählten Elektroden oder mit allen der Elektroden des Katheters 5, um so vor, zwischen, während und nach einzelnen Koagulationsvorgängen oder nach der Behandlung lokale Aussagen über die Herztätigkeit und somit auch über das Reizleitungsverhalten zu gestatten. Eine neutrale, am Patienten gut leitfähig angeordnete Bezugs- oder Neutral-Elektrode 17, die auch als indifferente Elektrode bezeichnet wird, legt hierbei ein elektrisches Bezugspotential für die erfindungsgemäße Vorrichtung 1 fest.

In weiterer und alternativer erfindungsgemäßer Ausgestaltung wird die Impedanz der Elektroden 6, 7, 8 in Bezug auf die Elektrode 17 als Maß für den Gewebekontakt erfasst, angezeigt und/oder zeitlich zugeordnet gespeichert. Dem behandelnden Arzt wird somit die Möglichkeit geboten, die korrekte Durchführung der Behandlung zu dokumentieren, oder zur Übung, beispielsweise im Tierversuch durchgeführte Behandlungsabläufe zu erfassen, auszuwerten und zu optimieren.

Ferner umfaßt die Vorrichtung 1 eine Bedieneinheit 18, mit welcher über ein Tastenfeld und/oder Pegel- oder Stellelemente die Betriebsparameter einstellbar sind. An einer Anzeigeeinrichtung 19, die ein oder mehrere numerische Anzeigefelder oder einen Anzeigeschirm aufweist, werden, vorzugsweise den jeweiligen Sensoren zugeordnet die momentane Betriebstemperatur und der zeitliche Verlauf bzw. die Zeitdauer der Zuführung von Hochfrequenzenergie angezeigt. Alternativ oder ergänzend werden entsprechende Balkendiagramme auf einem durch eine Schnittstelle 20 mit der Vorrichtung 1 verbundenen Personalcomputer 21 angezeigt. Mittels geeigneter Programme können die Behandlungsparameter auf einem Speichermedium abgelegt sowie später wieder zurückgewonnen werden.

Beispielhaft sei nachfolgend der Funktionsablauf der Vorrichtung 1 sowie ein Behandlungsablauf dargestellt.

Mittels der Bedieneinheit 18 und/oder dem Personalcomputer 21 lassen sich die behandlungsnotwendigen Betriebsparameter, wie beispielsweise Anzahl und Nummer der jeweils angesteuerten Elektroden, zeitliche Dauer der Ansteuerung und/oder Grenztemperatur eingeben.

Während des Behandlungsablaufs werden bei in das Herz des Patienten eingeführtem Katheter 5 durch die Steuereinheit 4 angesteuert selektiv die Elektroden 6, 7 und 8 mit Hochfrequenzenergie versorgt. Hierbei wird die momentane Temperatur je nach Betriebszustand der Vorrichtung durch die thermischen Sensoren 12, 13 und 14 teilweise, vollständig oder nicht erfaßt und gegebenenfalls der Steuereinheit 4 zugeführt. Die thermischen Sensoren können Thermistoren, Thermokappen, Thermokoppler und/oder Pelletier-Elemente sowie sonstige für medizinische Anwendungen geeignete Sensoren umfassen.

Bei einer Temperatur in einem Temperaturbereich von zwischen etwa 40 bis etwa 80°C, vorzugsweise bei 45, 50, 55 oder 60°C, werden im Herzen lokale Koagulationsnarben mit einer Tiefe von etwa 3 bis 5 mm erzeugt, wobei die Herzfunktion am EKG-Monitor 16 überwacht werden kann und alternativ die Impedanz einiger ausgewählter oder aller Elektroden 6, 7 und 8 angezeigt und aufgezeichnet werden kann.

Während Zeiten, innerhalb welcher keine der Elektroden 6, 7 und 8 oder nur ausgewählte Elektroden angesteuert werden, kann der HF-Generator 3 oder das handelsübliche Hochfrequenz-Ablationsgerät 3 mit einer Last 22, die vorzugsweise einen gekühlten Widerstand umfaßt, verbunden werden, um das Gerät 3 in einem stabilen, eingeschwungenen Zustand betreiben zu können, so daß Leistungsschwankungen vermieden werden. Hierbei entspricht der Wert der Lastimpedanz in etwa der Impedanz des zu behandelnden Gewebes und wird das Umschalten sowohl zu den Elektroden 6, 7 und 8 als auch zur Last 22 symmetrisch zum Bezugspotential der indifferenten Elektrode vorzugsweise mit Leistungs-FET's durchgeführt.

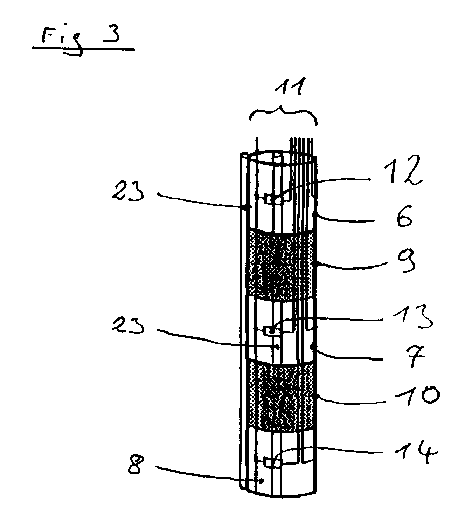

In Figur 3, umfasst ein Katheter 5 einen oder mehrere Lichtleiter 23, der oder die jeweils im aktiven Katheterbereich linienförmig Licht emittieren. Um eine definierte Lichtenergieabgabe in das Gewebe zu erreichen ist der oder sind die Lichtleiter 23 am Außen- oder Innenumfang des Katheters 5 angeordnet und gestatten unter Auswertung der simultan erfassten Impedanzwerte die thermische Koagulation mittels lichtfrequenter Engergie. Der Lichtleiter kann entweder ein sich über die gesamte aktive Katheterlänge erstreckendes Austrittsfenster aufweisen oder es können verschiedene Austrittsfenster mehrerer Lichtleiter ähnlich wie die Elektroden 6, 7 oder 8 angeordnet sein, um eine bereichsweise Steuerung zu ermöglichen. Um stets einen sicheren Kontakt zum Gewebe zu erreichen, können mehrere sich in Längsrichtung parallele zum Katheters erstreckende Lichtleiter an dessen Außen- oder Innenumfang angeordnet sein.

Der fortschreitende Behandlungserfolg ist mit dem EKG-Monitor 16 sowie durch beliebige weitere, für Fachleute auf dem Gebiet bekannte Überwachungs- oder Anzeigegeräte feststellbar und kann den behandelnden Arzt sicher leiten. Mit der erfindungsgemäßen Vorrichtung können in vorteilhafter Weise bereits vorhandene Behandlungsgeräte, wie beispielsweise HF-Generatoren 3, Hochfrequenz- Ablationsgeräte 3, EKG-Monitore 16 oder bei der letzgenannten Ausführungsform vorhandene Lasergeräte zusammen mit dem erfindungsgemäßen Katheter 5 eingesetzt und können übermäßig hohe Anschaffungskosten vermieden werden.

Die Elektroden 6, 7 und/oder 8 können weiterhin mit einem Gerät zur bipolaren oder unipolaren elektrischen Stimulation des Herzmuskels verbunden werden und auf diese Weise durch Stimulation des Herzens zur Erfassung der Erfolge des Behandlungsverlaufs beitragen.

Darüber hinaus ist die Erfindung nicht auf die vorteilhafte aktive Katheterlänge von etwa 7 cm bei einer Elektrodengröße von 4 mm und einer Isolationsbereichgröße von 3 mm beschränkt. An die jeweiligen Behandlungsaufgaben angepaßte, abweichende Größen sind für den Fachmann einfach zu verwirklichen oder können durch Auswahl der Anzahl der benutzten Elektroden 6, 7 oder 8 eingestellt werden. Auch die dargestellten Anzeige- und Datenaufzeichnungs- sowie - wiedergabegeräte sind lediglich beispielhaft, da prinzipiell beliebige bildgebende sowie speichernde Einrichtungen verwendbar sind.

## Patentansprüche

1. Hochfrequenz-Ablationskatheter zur linienförmigen endomyokardialen Katheterablation, umfassend mehrere durch Isolationsbereiche (9, 10) voneinander getrennte Elektroden (6, 7, 8), die selektiv ansteuerbar sind, um vorzugsweise endomyokardiales Gewebe zu koagulieren, wobei den Elektroden (6, 7, 8) jeweils thermische Sensoren (12, 13, 14) so zugeordnet sind, daß die Betriebstemperatur der einen oder mehreren der Elektroden erfaßbar und in Zusammenwirken mit einer Steuereinheit (4), die programmiert ist, um in Abhängigkeit von der gewählten Zuordnung der thermischen Sensoren in einer von mehreren Betriebsarten zu arbeiten, in deren zeitlichem Verlauf und deren absoluter Höhe einstellbar ist.

2. Hochfrequenz-Ablationskatheter nach Anspruch 1, ferner gekennzeichnet durch die Verbindung (11) einer oder mehrerer Elektroden (6, 7, 8) zu einem EKG-Monitor (16).

3. Hochfrequenz-Ablationskatheter nach Anspruch 1 oder 2, weiter umfassend einen oder mehrere Lichtleiter (23) mit linienförmigen Emissionszentren.

4. Vorrichtung zur Hochfrequenz-Katheterablation endomyokardialen Gewebes, umfassend einen Ablationskatheter (5) nach einem der Ansprüche von 1 bis 3, wobei die Steuereinheit entweder einen diesem zugeordneten Anschluß (2, 11) an einen Hochfrequenzgenerator (3) besitzt oder ein Hochfrequenzablationsgerät (3) umfaßt.

5. Vorrichtung nach Anspruch 4, dadurch gekennzeichnet, daß die Steuereinheit so programmiert ist, daß die Elektroden (6, 7, 8) für das Durchführen eines lokalen Koagulationsvorgangs jeweils eine in einem Bereich von zwischen 40 bis 80 Grad Celsius, vorzugsweise bei 45, 50, 55 oder 60 Grad Celsius liegende, festlegbare Endtemperatur erreichen.

6. Vorrichtung nach Anspruch 4 oder 5, dadurch gekennzeichnet, daß die Steuereinheit so programmiert ist, daß den Elektroden (6, 7, 8) zyklisch oder statistisch Energie zugeführt wird und nach Erreichen der Endtemperatur die Energie vom Hochfrequenzgenerator (3) oder Hochfrequenzablationsgerät (3) durch die Steuereinheit (4) vermindert oder vorzugsweise der nächsten Elektrode (6, 7, 8) zugeführt wird.

7. Vorrichtung nach Anspruch 4 oder 5, dadurch gekennzeichnet, daß die Steuereinheit so programmiert ist, daß den Elektroden (6, 7, 8) Energie in Form von elektromagnetischen Impulsen mit fester oder variabler Länge und/oder fester oder variabler Wiederholungsrate zugeführt wird.

8. Vorrichtung nach einem der Ansprüche 4 bis 7, dadurch gekennzeichnet, daß die Steuereinheit so programmiert ist, daß die vom Hochfrequenzgenerator (3) oder vom Hochfrequenzablationsgerät (3) gelieferte, jedoch momentan nicht benötigte Leistung an eine Last (22), insbesondere an einen oder mehrere Widerstände, die jeweils gekühlt werden, abgeleitet wird.

9. Vorrichtung nach einem der Ansprüche 4 bis 8, dadurch gekennzeichnet, daß die Elektroden (6, 7, 8) entlang einer ununterbrochenen Linie mit einer Länge von bis zu etwa 7 cm jeweils durch Isolationsbereiche (9, 10) voneinander getrennt, vorzugsweise am Ende des Ablationskatheters (5), angeordnet sind.

10. Vorrichtung nach einem der Ansprüche 4 bis 9, dadurch gekennzeichnet, daß die Temperatur einer jeweiligen Elektrode (6, 7, 8) und die Zeitdauer der Energieabgabe an diese Elektrode (6, 7, 8) an zumindest einer Anzeigeeinrichtung (19, 21) darstellbar sind.

11. Vorrichtung nach einem der Ansprüche 4 bis 10, dadurch gekennzeichnet, daß mittels einer Verbindung (11) von einer oder mehreren Elektroden (6, 7, 8) zu einem EKG-Monitor (16) die lokale Herztätigkeit vor, zwischen, während und nach der Behandlung erfaßbar und anzeigbar ist.

12. Vorrichtung nach einem der Ansprüche 4 bis 11, dadurch gekennzeichnet, daß an einem Bedienfeld (18) die Anzahl der zur Ablation benötigten Elektroden (6, 7, 8), deren Solltemperatur und/oder zeitliche Energiezufuhr und/oder der Betriebszustand einstellbar ist.

## Claims

1. High-frequency ablation catheter for linear endomyocardial catheter ablation, including several electrodes (6, 7, 8) which are separated from each other by isolation zones (9, 10) and which can be controlled selectively to preferably coagulate endomyocardial tissue, the electrodes (6, 7, 8) each being assigned thermal sensors (12, 13, 14) such that the operating temperature of one or more of the electrodes can be detected and, in cooperation with a control unit (4) which is programmed to work in one of several modes depending on the selected coordination of the thermal sensors, can be adjusted in its time progression and its absolute quantity.

2. High-frequency ablation catheter according to claim 1, further characterised by the link (11) of one or more electrodes (6, 7, 8) to an ECG monitor (16).

3. High-frequency ablation catheter according to claim 1 or 2, further including one or more fibre optic light guides (23) with linear emission centres.

4. Apparatus for high-frequency catheter ablation of endomyocardial tissue, including an ablation catheter (5) according to any of claims 1 to 3, wherein the control unit either has a connection (2, 11) to a high-frequency generator (3) associated with the catheter (5) or includes a high-frequency ablation device (3).

5. Apparatus according to claim 4, characterised in that the control unit is programmed in such a way that the electrodes (6, 7, 8) for carrying out a local coagulation process each reach a definable final temperature which is within a range from between 40 to 80 degrees Celsius, preferably at 45, 50, 55 or 60 degrees Celsius.

6. Apparatus according to claim 4 or 5, characterised in that the control unit is programmed in such a way that energy is supplied to the electrodes (6, 7, 8) cyclically or statistically and on reaching the final temperature the energy from the high-frequency generator (3) or high-frequency ablation device (3) is reduced by the control unit (4) or preferably supplied to the next electrode (6, 7, 8).

7. Apparatus according to claim 4 or 5, characterised in that the control unit is programmed in such a way that energy is supplied to the electrodes (6, 7, 8) in the form of electromagnetic pulses of fixed or variable length and/or fixed or variable repetition rate.

8. Apparatus according to any of claims 4 to 7, characterised in that the control unit is programmed in such a way that the power which is delivered by the high-frequency generator (3) or by the high-frequency ablation device (3) but not needed for the moment is conducted away to a load (22), in particular to one or more resistors which are each cooled.

9. Apparatus according to any of claims 4 to 8, characterised in that the electrodes (6, 7, 8) are arranged in a continuous line with a length of up to about 7 cm, each separated from the other by isolation zones (9, 10), preferably at the end of the ablation catheter (5).

10. Apparatus according to any of claims 4 to 9, characterised in that the temperature of a respective electrode (6, 7, 8) and the duration of energy delivery to this electrode (6, 7, 8) can be displayed on at least one display device (19, 21).

11. Apparatus according to any of claims 4 to 10, characterised in that by means of a link (11) from one or more electrodes (6, 7, 8) to an ECG monitor (16) the local heart activity before, between, during and after treatment can be detected and displayed.

12. Apparatus according to any of claims 4 to 11, characterised in that on a control panel (18) the number of electrodes (6, 7, 8) needed for ablation, whose nominal temperature and/or energy supply in time and/or the operating state is adjustable.

## Revendications

1. Cathéter d'ablation haute fréquence pour l'ablation linéaire par cathéter endomyocardique, comprenant plusieurs électrodes (6, 7, 8), séparées les unes des autres par des zones d'isolation (9, 10), électrodes susceptibles d'être commandées sélectivement, pour faire coaguler de préférence des tissus endomyocardiques, des capteurs thermiques (12, 13, 14) étant respectivement associés aux électrodes (6, 7, 8), de manière que la température de fonctionnement d'une ou plusieurs des électrodes puisse être appréhendée et, en coopération avec une unité de commande (4) qui est programmée, puisse être réglée en l'évolution temporelle et en hauteur absolue, pour travailler en un parmi plusieurs types de fonctionnement, en fonction de l'association sélectionnée des capteurs thermiques.

2. Cathéter d'ablation haute fréquence selon la revendication 1, caractérisé en outre par la liaison (11) d'une ou plusieurs électrodes (6, 7, 8) à un moniteur d'électrocardiogramme ECG (16).

3. Cathéter d'ablation haute fréquence selon la revendication 1 ou 2, comprenant en outre un guide de lumière équipé de centres d'émission à forme linéaire.

4. Dispositif d'ablation par cathéter haute fréquence de tissus endomiocardiques, comprenant un cathéter d'ablation (5), selon l'une des revendications 1 à 3, l'unité de commande comportant soit un raccordement (2, 11), associé à celui-ci, à un générateur haute fréquence (3), soit un appareil d'ablation haute fréquence (3).

5. Dispositif selon la revendication 4, caractérisé en ce que l'unité de commande est programmée de manière que les électrodes (6, 7, 8), destinées à la mise en oeuvre d'un processus de coagulation local, atteignent chacune une température finale susceptible être fixée, située dans une plage comprise entre 40 à 80 degrés Celsius, de préférence à 45, 50, 55 ou 60 degrés Celsius.

6. Dispositif selon la revendication 4 ou 5, caractérisé en ce que l'unité de commande est programmée de manière que de l'énergie soit amenée, de façon cyclique ou statistique, aux électrodes (6, 7, 8) et que, après atteinte de la température finale, l'énergie venant du générateur haute fréquence (3) ou de l'appareil d'ablation à haute fréquence (3) soit diminuée au moyen de l'unité de commande (4) ou, de préférence, soit amenée à l'électrode suivante (6, 7, 8).

7. Dispositif selon la revendication 4 ou 5, caractérisé en ce que l'unité de commande est programmée de manière qu'aux électrodes (6, 7, 8) soit amenée de l'énergie se présentant sous la forme d'impulsions électromagnétiques, ayant une longueur fixe ou variable et/ou un taux de répétition fixe ou variable.

8. Dispositif selon l'une des revendications 4 à 7, caractérisé en ce que l'unité de commande est programmée de manière que la puissance, fournie par le générateur haute fréquence (3) ou par l'appareil d'ablation à haute fréquence (3), qui cependant n'est momentanément pas nécessaire, est dérivée sur une charge (22), en particulier sur une ou plusieurs résistances, chacune étant refroidie.

9. Dispositif selon l'une des revendications 4 à 8, caractérisé en ce que les électrodes (6, 7, 8) sont disposées le long d'une ligne ininterrompue, d'une longueur allant jusqu'à environ 7 cm, séparées les unes des autres au moyen de zones d'isolation (9, 10), de préférence à l'extrémité du cathéter d'ablation (5).

10. Dispositif selon l'une des revendications 4 à 9, caractérisé en ce que la température d'une électrode (6, 7, 8) respective et la durée de fourniture d'énergie à cette électrode (6, 7, 8) sont susceptibles être affichées en au moins un dispositif d'affichage (19, 21).

11. Dispositif selon l'une des revendications 4 à 10, caractérisé en ce qu'au moyen d'une liaison (11), allant d'une ou plusieurs électrodes (6, 7, 8) à un moniteur d'électrocardiogramme (16), on peut appréhender et afficher l'activité cardiaque avant, entre, pendant et après les traitements.

12. Dispositif selon l'une des revendications 4 à 11, caractérisé en ce que le nombre d'électrodes (6, 7, 8) nécessaires pour l'ablation, leur température de consigne et/ou l'apport temporel en énergie et/ou l'état de fonctionnement peuvent être réglés sur un tableau de commande (18).
